# EUROPEAN PATENT APPLICATION

(11) **EP 4 567 102 A1**
(43) Date of publication of application: **11.06.2025**
(21) Application number: 23307141.4
(22) Date of filing: 05.12.2023
(51) Int. Cl.: C12N 5/0735

(54) **METHODS FOR ISOLATING EMBRYONIC STEM CELLS FROM AVIAN EMBRYONIC CELLS**

(71) Applicant: Suprême, 91058 Evry-Courcouronnes Cedex (FR)
(72) Inventor: LÓPEZ DÁVILA, Víctor, 91058 Evry-Courcouronnes Cedex (FR); LONG-MARTEL, Marianne, 91058 Evry-Courcouronnes Cedex (FR)
(74) Representative: A.P.I. Conseil

(57) **Abstract**

The invention relates to a process for isolating embryonic stem cells (100) from at least one avian embryonic cells comprising: isolating at least one embryo (101) at a developmental stage around oviposition; suspending embryonic cells (102) obtained by dissociating embryo(s) of step a) in an animal serum free basal culture medium supplemented with a combination of growth factors, at least one inhibitor and a substitute to animal serum; seeding the suspension of embryonic cells (103) on a layer of feeder cells ; culturing (104) the embryonic cells for at least one passage.

## Description

### FIELD OF THE INVENTION

The invention belongs to the field of biotechnology. More particularly, the invention relates to a process of isolating embryonic stem cells from avian embryos and to kits and media useful to implement the methods of the invention.

### BACKGROUND OF THE INVENTION

Embryonic stem cells (ESCs) are characterized by their ability to differentiate into any cell type and by their ability to self-renew. Such cells provide biotechnical tools of an utmost interest, as well as promising candidates in the medical field; because of their ability to differentiate into a variety of cell types. ESCs find application in tissue engineering, replacement therapy, drug discovery, vaccine production, cultivated leather etc. Furthermore, facing the expected increase in worldwide meat consumption associated with the booming of cultivated meat technologies, the use of ESCs in methods for producing cultivated meat is of particular interest notably because of the possibility to obtain large amounts of cells of various lineages starting from only one cell line.

Several meat substitutes have been developed from insects, vegetable components and/or cultured animal, fungus or vegetable cells (i.e. cell technology). In particular, cell technologies are being rapidly developed to respond to new consumer demand.

WO 2003/076601, WO 2008/129058, WO 2020/104650 disclose methods for producing avian cell lines which comprise a step of isolating ESCs. Nevertheless, these methods suffer from lack of reproducibility that might, at least in part, rely on the need of animal-originating compounds and media that are subjected to uncontrollable variations in their composition. There is thus a need for a reproducible method of isolating ESCs.

The applicant has been able to develop a reproducible process of isolating ESCs of avian origin limiting the use of undefined compounds and/or media of animal origin and, incidentally, therefore complies with the growing concern in many societies of preserving animal life and welfare, avoiding animal exploitation and slaughtering.

### SUMMARY

Invention relates, *inter alia,* to a process of isolating ESCs from at least one avian embryo. Inventors have been able to define specific combinations of growth factors that, combined with other specific culture conditions, allows a reproducible isolation of avian ESCs. Furthermore, implementation of said process limits the use of compounds of animal origin and does not use media of undefined composition.

Accordingly, in a first aspect, the invention relates to a process for isolating embryonic stem cells (100) from at least one avian embryo comprising the steps of :
a. isolating at least one embryo at a developmental stage around oviposition,
b. suspending embryonic cells obtained by dissociating embryo(s) of step a) in an animal serum free basal culture medium supplemented with :
   - a mix of growth factors which modulates at least the following pathways : JAK/STAT, PI3K/AKT, SHP2/MAPK, PLC-gamma, MAPK, PI3K/AKT/MTOR, RAS/RAF, RHOA/ROCK
   - at least one inhibitor leading to the inhibition of MEK-signalling,
   - at least one inhibitor leading to the inhibition of Wnt-signalling, and
   - a substitute to animal serum,
c. seeding the suspension of embryonic cells obtained in step b) on a layer of feeder cells,
d. culturing (104) the embryonic cells for at least one passage.

Modulations of these pathways are sufficient to allow maintenance of the pluripotency properties of the cells and culture conditions set by the inventor and allow a low attrition rate.

According to other optional features of the process according to the invention, it can optionally include one or more of the following characteristics alone or in a combination:
- the mix of growth factors comprises at least one ortholog of the following growth factors : interleukin 6 (IL6), Leukemia inhibitory factor (LIF), Insulin Growth factor 1 (IGF-1), and Stem Cell Factor (SCF),
- IL6 is selected from avian IL6 or mammalian IL6, or a mix thereof, LlF is selected from avian LIF or mammalian LIF, or a mix thereof, IGF-1 is selected from avian IGF-1 or mammalian IGF-1, or a mix thereof, or SCF is selected from avian SCF or mammalian SCF, or a mix thereof,
- the process further comprising at least one inhibitor leading to the inhibition of PKC-signalling,
- the inhibitor of MEK- signalling is selected from a small molecule or a MEK-1 siRNA, the inhibitor of Wnt-signalling is selected from a small molecule or a Wnt-1 siRNA, or the inhibitor of PKC- signalling, when present, is selected from a small molecule or a PKC-alpha siRNA
- the inhibitor leading to the inhibition of MEK-signalling is selected from : binimetinib, cobimetinib, selumetinib, trametinib, CI-1040, PD035901, TAK-733, PD-325901, PD98059 or U0126, or a mix thereof, the inhibitor leading to the inhibition of Wnt-signalling is selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004, or the inhibitor leading to the inhibition of PKC-signalling, when present, is selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, or midostaurin, or a mix thereof.,
- the animal serum substitute, is selected from : KnockOut^{™} serum replacement, Serum Replacement Basic ; Serum Replacement 3 ; BIT 9500 Serum Substitute from StemCell,
- avian embryonic cells are chicken embryonic cells or duck embryonic cells.

In a second aspect, the invention relates to a process for obtaining a continuous diploid cell line derived from avian embryonic stem cells (ESCs) comprising :
a. providing at least one avian ESC obtained from the process for isolating embryonic stem cells from avian embryo according to the invention,
b. progressively withdrawing each of the growth factors from the culture medium, progressively withdrawing the at least one inhibitor from culture medium, progressively decreasing the concentration of feeder cells in the culture so as to obtain a total withdrawal of the feeder layer after several passages,
thereby obtaining adherent or non-adherent continuous diploid avian cell lines derived from avian ESCs capable of proliferating in a basal medium in the absence of growth factors.

The highly reproducible and reliable process of isolating ESCs, allows to simply obtain a cell line derived thereof, which is of use in many fields ranging, e.g., from biotechnology and research, medicine (e.g. vaccine production) to cultivated meat.

In a third aspect, the invention relates to a kit of parts adapted for reconstituting a cell culture medium comprising:
- an ortholog of interleukin 6 (IL6),
- an ortholog of Leukemia inhibitory factor (LIF),
- an ortholog of Insulin Growth factor 1 (IGF-1),
- an ortholog of Stem Cell Factor (SCF),
- at least one inhibitor of MEK signalling selected from i) a small molecule selected from binimetinib, cobimetinib, selumetinib, trametinib, CI-1040, PD035901, TAK-733, PD-325901, or a mix thereof, or ii) a MEK-1 siRNA,
- at least one inhibitor of Wnt signalling is selected from i) a small molecule selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004or a mix thereof or ii) a Wnt-1 siRNA.

According to an optional feature said kit further comprises at least one inhibitor of PKC signalling selected from i) a small molecule selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin or a combination thereof or ii) a PKC-alpha siRNA.

According to another optional feature, said kit further comprises an animal serum substitute.

### FIGURE LEGENDS

Figure 1: Scheme of the steps of a process 100 of isolating embryonic stem cells according to the invention.
Figure 2: Scheme of the steps of a process 200 of obtaining a continuous diploid cell line.
Figure 3: Pluripotency assays performed for duck ESCs isolated through the process of the invention. A: results of RT-qPCR assay measuring expression pluripotency marker genes after 5 passages of the cells ; expression level (arbitrary units) in ESCs (black) are compared to expression levels in Stage X Embryo (grey) and in duck fibroblast; none of the tested genes is detectably expressed in fibroblasts. B: Immunofluorescence for SSEA1 and EMA1 after 20 passages of the ESCs isolated through the process of the invention.

### DETAILED DESCRIPTION AND ADDITIONAL EMBODIMENTS

### Definitions

As used herein, "Embryonic stem cells" (ESCs) relate to undifferentiated cells that have the capacity to self-renew by dividing and to develop into the three primary germ cell layers (namely, the endoderm, the ectoderm, and the mesoderm) and their derivatives. In vertebrates, almost all cell types constituting the animal at birth originate from a subset of transiently pluripotent cells called the inner cell mass (ICM) in the mammalian blastocyst-stage pre-implantation embryo or the blastoderm (BDM) in oviparous vertebrates (birds, reptiles, amphibians, fishes). BDM cells can also easily be isolated from early oviparous vertebrate embryos, to create pluripotent ESC lines (WO 03/076601, WO 2008/129058, WO 2020/104650). Avian BDM cells suitable for generating ESCs can be obtained from any bird species.

As used herein, "avian" means to relate to any bird. "Bird" refers to any species, subspecies or race of organisms of the *ava* taxonomic class. In particular a bird can belong to the following orders:
- order of "Anseriformes". This order gathers mainly aquatic birds. It comprises three families (the *Anhimidae, Anseranatidae, Anatidae*) with around 170 species and 51 genera of ducks, geese, screamers, magpie geese and swans. Most species are highly adapted to aquatic environment;
- order of "Galliformes" (e.g. chicken, quails, turkey, pheasant and allies). Galliformes order includes 5 families *Phasianidae* (including chicken, quail, partridges, pheasants, turkeys, peafowl (peacocks) and grouse), *Odontophoridae* (New World quail), *Numididae* (guinea fowl), *Cracidae* (including chachalacas and curassows), and *Megapodiidae* (incubator birds like malleefowl and brush-turkeys), accounting for around 301 species;
- "Columbiformes" (i.e Pigeon and allies). This order includes only one family, that includes doves and pigeons, accounting for 344 species;
In another particular embodiment avian bird refers to a poultry selected from, but not restricted to: chicken, turkey, duck, goose, guinea fowl, pigeon, quail, squab or even pheasant, emu, swan, ostrich, parrots, finches, hawks, crows, and cassowary.

As used herein, "chicken" refers to the species *Gallus domesticus;* "duck" refers to any duck belonging to the biological order of Anseriformes. In a particular embodiment "duck" refers to the species *Anas platyrhynchos* (Pekin duck) or to a hybrid of the species *Cairina moschata domestica* and *Anas platyrhynchos domesticus* (Mulard duck).

As used herein, "passage" when related to the field of cell culture refers to the transfer of cultured cells, with or without intended dilution, from one culture vessel to another. The passage number is the number of times the cells in culture in adherence have been passed in a new vessel. Cells can be passed or subcultured at any time. Usually, cells are passed when they reach confluency (for adherent cells) and/or a given density in the culture medium. Passage can basically occur at any time, as a function of doubling time of the cells, stability of medium components, stability of the feeder layer, at predetermined time intervals and so on, provided that cell density is sufficient to ensure cell culture restarts properly. Passage time can be chosen as a function of the size of the colonies, typically a passage can be done when colonies reach 100-150 µm diameter, ESCs isolated following the method of the invention can be subsequently submitted to specific culture steps in order to obtain cell lines that are able to be cultured over an extended period of time and/or number of passages, even continuously, while keeping their pluripotency, undifferentiated state and viability.

As used herein, "small molecule" refers to, according to the usual meaning in molecular biology and pharmacology, a small molecule (an organic compound) of a molecular weight equal or lower than 1000 daltons that modulates a biological process, leading to a biological effect. For instance, polymeric molecules as nucleic acids, proteins or polysaccharides are not small molecules in the meaning of this invention, whereas ribo- or deoxyribonucleotides, amino acids, or monosaccharides are considered as small molecules.

In the context of the present invention, reference to a specific drug or compound includes not only the specifically named drug or compound, but also any pharmaceutically acceptable salt, hydrate, derivative, isomer, racemate, enantiomerically pure composition, conjugate or corresponding prodrug of the active molecule of the drug or of said compound. Preferably, reference to a compound includes the specifically named compound, as well as any pharmaceutically acceptable salt, hydrate, isomer, racemate, isomer, or enantiomerically pure composition of said compound. More preferably, the designation of a compound is intended to designate the compound as specifically designated *per se,* as well as any pharmaceutically acceptable salt thereof.

As used herein, "ortholog" when used for growth factors used in the process according to the invention refers to any protein of any organism that is thought to perform a function equivalent to that of growth factors as herein specifically named. Orthologs can be easily retrieved in genome databases as e.g., Ensembl (<http://www.ensembl.org/index.html>) or other specialized databases well known in the art. Noteworthy, a high level of conservation of the genetic pathways and morphogenetic mechanisms governing embryonic development is observed in vertebrates from fishes to humans. In other words, a growth factor used in the process according to the invention can correspond in its sequence to a growth factor of any origin, of mammalian (e.g. human or mouse) or of avian organism, provided that it performs a function equivalent to that of growth factors as herein specifically named. Accordingly, in a particular embodiment an ortholog for a growth factor useful in a process of the invention encodes a protein whose sequence shares at least 30% homology in its amino acid sequence with the corresponding protein in chicken (*Gallus domesticus*) or duck (*Anas platyrhynchos*), preferably, more than 30%, preferably 31%, 32%, 33%, 34%, 35%, 36%, 37%, 38%, or even 39%, preferably said sequence share is at least of 40%, preferably more than 40%, preferably 41%, more preferably 42%, 43%, 44%, 45%, 46%, 47%, 48%, even more preferably 49%, preferably said sequence share is at least of 50%, preferably more than 50%, preferably 51%, 52%, 53%, 54%, 55%, 56%, 57%, 58%, even more preferably 59%, preferably said sequence share is at least 60%, preferably more than 60%, preferably 61%, 62%, more preferably 63%, 64%, 65%, 66%, 67%, 68%, even more preferably 69%, preferably said sequence share is at least 70%, preferably more than 70%, preferably 71%, 72%, 73%, 74%, 75%, 76%, 77%, 78%, even more preferably 79%, preferably, said sequence share is at least 80%, preferably more than 80%, preferably 81%, 82%, 83%, 84%, 85%, 86%, 87%, 88%, even more preferably 89%, preferably said sequence share is at least 90%, preferably more than 90%, preferably 91%, 92%, 93%, 94%, 95%, 96% 97%, 98% and even more preferably more than 99% of homology.

A first object of the invention is the process the inventors have developed that allows to isolate ESCs from avian embryos. Said process is reproducible, with a high rate of success and does not involve compounds and/or media of animal origin nor of undefined composition.

### Process of isolating avian embryonic stem cells.

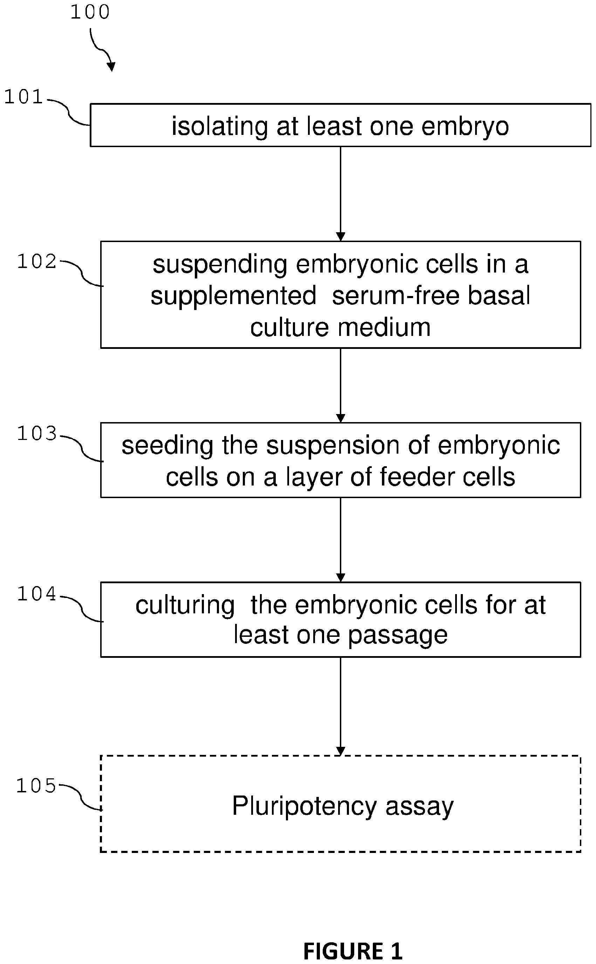

As illustrated figure 1 and as mentioned above, an object of the invention relates to a process of isolating embryonic stem cells 100 from an avian embryo comprising the following steps of :
a. isolating at least one embryo 101 at a developmental stage around oviposition,
b. suspending embryonic cells 102 obtained by dissecting embryo(s) of step a) in an animal serum-free basal culture medium supplemented with :
   - a mix of growth factors which modulates at least the following pathways : JAK/STAT, PI3K/AKT, SHP2/MAPK, PLC-gamma, MAPK, PI3K/AKT/MTOR, RAS/RAF, RHOA/ROCK,
   - at least one inhibitor leading to the inhibition of MEK-signalling,
   - at least one inhibitor leading to the inhibition of Wnt-signalling, and
   - an animal serum substitute,
c. seeding the suspension of embryonic cells 103 obtained in step b) on a layer of feeder cells,
d. culturing 104 the embryonic cells for at least one passage.

### Step a) of isolating at least one embryo 101 at a developmental stage around oviposition

Preferably, for the purpose of the process 100 according to the invention, the egg is less than 2 days old, indeed, embryonic cells will progressively differentiate from the time of laying and it is preferable to have a content in differentiated cells as low as possible.

The egg can come from any bird as defined above. In a preferred embodiment, the at least one egg is a duck or a chicken egg.

Obviously, the process 100 of the invention can comprise prior steps of providing at least one fertilized egg, and preferably, of carefully washing it first with a detergent solution. A further prior step can comprise, before breaking the at least one egg, washing the egg with a 70% ethanol solution.

In a particular embodiment, in order to block the embryo's development, eggs can be stored between 18-21°C for 0 to 3 days, or between 15-17°C for 4 to 7 days, or between 10-12°C for 8 to 10 days before the embryo isolation step.

As specified, in the process 100 of the invention the avian ESCs are collected from an avian embryo around oviposition, from a laid fertilized egg. Oviposition corresponds to different development stages depending on the species from which the egg originates. These stages are well known from the skilled person in the art and described in either Eyal-Giladi and Kochan (1976) or Sellier et al. (2006). Basically, depending on the species, it can range from stage VII to stage XI.

For example, and in a particular embodiment, for muscovy duck a suitable stage is stage VII, for guinea fowl a suitable stage is stage VII - VIII, for turkey a suitable stage is stage VII-VIII, for pekin duck a suitable stage is stage VIII, for chicken a suitable stage is stage X, for japanese quail a suitable stage is stage XI, for goose a suitable stage is stage XI.

Any method of isolating an embryo from an egg can be applied in order to isolate the embryo. Such methods are well known in the art. In a particular embodiment, the isolating step 101 can comprise the following sub-steps:
i. carefully breaking the egg to keep the yolk intact,
ii. separating the white from the yolk and placing the yolk with the embryo on the top in a vessel suitable for maintaining the yolk to allow further dissection. For example, 5 yolks can be maintained in e.g. a 14 cm Petri-dish,
iii. removing the excess of white on the surface of the embryo using sterilized Whatman^{®} papers,
iv. placing a new sterilized Whatman^{®} paper in which disc has been precut with a diameter of 0.4 cm to 1.6 cm in such a way to have the embryo placed in the center of the disc and capillary adhering to the disc,
v. quickly retrieving the paper carrying the embryo from the yolk,
vi. removing as much yolk as possible from the paper and placing face up the disc (embryo upward) in e.g. a new 14 cm dish previously filled with room temperature PBS,
vii. separating the epiblast region from zona pellucida, and gathering zona pellucida that has a high content in stem cells.

Optionally in step iv, four small incisions are made in the precut, outside the paper disk, with a sterile scalpel and sterile forceps are used to retrieve the embryo.

The diameter of the disc which is precut at step iv is supposed to be larger than the embryo in order to allow its easy collecting, but not too large in order to facilitate manutention and avoid contaminating the embryo with too much yolk. Otherwise said, diameter is comprised between 0.4 cm and 1.6 cm, preferably 0.6 cm and 1.2 cm. In a particular embodiment said diameter is of around 0.8 cm. The person skilled in art will know how to adapt the diameter of the disc according to the size of the embryo, which can vary according to the bird species.

In step vi, any method can be used to get off as much yolk as possible from the embryo and to clean it, for example,
- using a 1 mL syringe with a 30G needle aspirate 0.5 mL of cold PBS, then with the needle isolating and cleaning the embryo as much as possible without losing it, or
- isolating and cleaning the embryo using the ring of an inoculation loop.

An alternative to steps iv-v can be using a micropipette with a 1000 µL tip cut to allow a suitable aspiration of the embryo directly from the yolk.

### Step b) suspending embryonic cells 102 obtained by dissecting embryo(s) of step a) in an animal serum-free basal culture medium supplemented with growth factors and inhibitors:

The inventors have found a specific culture condition that allows to properly isolate and maintain ESCs in an undifferentiated state. These culture conditions are the combination of the use, in the culture medium, of
- a mix of growth factors that activates JAK/STAT, PI3K/AKT, SHP2/MAPK, PLC-gamma, MAPK, PI3K/AKT/MTOR, RAS/RAF and RHOA/ROCK pathways,
- a mix of inhibitors that aims to block MEK- and Wnt-signalling,
- an animal serum-free medium.

Of note there's no need, in the process according to the invention to chemically (e.g. using divalent ions chelators like E.D.T.A.) and/or enzymatically dissociate cells of zona pellucida (e.g. trypsin or any other convenient enzyme) collected in step vii, which is a further advantage in regard to the methods of the art.

Examples of suitable growth factors to be used in the mix of growth factors are listed in table 1 below.

**Table 1**

| **Growth factor** | **Uniprot^{†} reference** | **Pathways activated** |
|---|---|---|
| duck LIF | A0A493U243 | JAK/STAT |
| | | PI3K/AKT |
| | | SHP2/MAPK |
| duck IL6 | U3IW75 | JAK/STAT |
| duck FGF2 | U3IH98 | RAS/MAP |
| | | PI3K/AKT |
| | | PLC-gamma |
| duck SCF | U3IVD8 | JAK/STAT |
| | | PI3K/AKT |
| | | PLC-gamma |
| | | MAPK |
| duck IGF-1 | U3IT44 | PI3K/AKT/MTOR |
| | | SHP2/MAPK |
| | | RAS/RAF |
| | | RHOA/ROCK |
| chicken LIF | A0A8V0X6M3 | JAK/STAT |
| | | PI3K/AKT |
| | | SHP2/MAPK |
| chicken IL6 | Q90YI0 | JAK/STAT |
| chicken FGF2 | P48800 | RAS/MAP |
| | | PI3K/AKT |
| | | PLC-gamma |
| chicken SCF | Q09108 | JAK/STAT |
| | | PI3K/AKT |
| | | PLC-gamma |
| | | MAPK |
| chicken IGF-1 | P18254 | PI3K/AKT/MTOR |
| | | SHP2/MAPK |
| | | RAS/RAF |
| | | RHOA/ROCK |
| human LlF | P15018 | JAK/STAT |
| | | PI3K/AKT |
| | | SHP2/MAPK |
| human IL6 | P05231 | JAK/STAT |
| human IL6-R | P08887 | JAK/STAT |
| human SCF | P21583 | JAK/STAT |
| | | PI3K/AKT |
| | | PLC-gamma |
| | | MAPK |
| human IGF-1 | P05019 | PI3K/AKT/MTOR |
| | | SHP2/MAPK |
| | | RAS/RAF |
| | | RHOA/ROCK |
| human IL11 | P20809 | ERK |
| | | STAT |
| human FGF2 | P09038 | RAS/MAP |
| | | PI3K/AKT |
| | | PLC-gamma |

In the process 100 of the invention any mix of growth factors that mainly allows an upregulation of JAK/STAT, PI3K/AKT, SHP2/MAPK, PLC-gamma, MAPK, PI3K/AKT/MTOR, RAS/RAF and RHOA/ROCK pathways can be used. Upregulating these pathways has been found useful to, during the process 100 of isolating ESCs of the invention, maintaining the cells in an undifferentiated state. As mentioned above a growth factor used in the process according to the invention can correspond in its sequence to a growth factor of any origin, of a mammalian (e.g. human or mouse) or of an avian organism, provided that it performs a function equivalent to that of growth factors as herein specifically named.

In a particular embodiment, any mix of growth factors selected from a duck LlF, a duck IL6, a duck FGF2, a duck SCF, a duck IGF-1, a chicken LlF, a chicken IL6, a chicken FGF2, a chicken SCF, a chicken IGF-1, human LIF, human IL6, human IL6-R, human SCF, human IGF-1, human IL11, human FGF2, or an ortholog thereof as defined above can be used.

A useful mix of growth factors that can modulate these pathways is a mix comprising at least one interleukin 6 (IL6), at least one leukemia inhibitory factor (LIF), at least one insulin growth factor 1 (IGF-1), and at least one stem cell factor (SCF), or an ortholog thereof as defined above.

Preferably, the growth factors are avian growth factors. Even more preferably, growth factors are from the same *Aves* species as the dissected embryo.

In a particular embodiment, the embryo is a chicken embryo and the mix of growth factors is any mix of growth factors selected from a duck LlF, a duck IL6, a duck FGF2, a duck SCF, a duck IGF-1, a chicken LlF, a chicken IL6, a chicken FGF2, a chicken SCF, a chicken IGF-1, human LIF, human IL6, human IL6-R, human SCF, human IGF-1, human IL11, human FGF2, an ortholog thereof as defined above. In a more particular embodiment, the embryo is a chicken embryo and the mix of growth factors is a mix of growth factors consisting in at least one chicken LIF, at least one chicken IL6, at least one chicken SCF, at least one chicken IGF-1. In another particular embodiment, the embryo is a chicken embryo and the mix of growth factors is a mix of growth factors consisting in at least one human LIF, at least one human IL6, at least one human SCF, at least one human IGF-1. In a further particular embodiment, the embryo is a chicken embryo and the mix of growth factors is a mix of growth factors consisting in at least one duck LIF, at least one duck IL6, at least one duck SCF, at least one duck IGF-1.

In a particular embodiment, the embryo is a duck embryo and the mix of growth factors is any mix of growth factors selected from a duck LlF, a duck IL6, a duck FGF2, a duck SCF, a duck IGF-1, a chicken LlF, a chicken IL6, a chicken FGF2, a chicken SCF, a chicken IGF-1, human LIF, human IL6, human IL6-R, human SCF, human IGF-1, human IL11, human FGF2, an ortholog thereof as defined above. In a more particular embodiment, the embryo is a duck embryo and the mix of growth factors is a mix of growth factors consisting of at least one chicken LIF, at least one chicken IL6, at least one chicken SCF, at least one chicken IGF-1. In another particular embodiment, the embryo is a duck embryo and the mix of growth factors is a mix of growth factors consisting of at least one human LIF, at least one human IL6, at least one human SCF, at least one human IGF-1. In a further particular embodiment, the embryo is a duck embryo and the mix of growth factors is a mix of growth factors consisting of at least one duck LIF, at least one duck IL6, at least one duck SCF, at least one duck IGF-1.

In a particular embodiment, the relative ratio of concentration of LIF/IL6/SCF/IGF-1 in the final culture medium of 10/10/1/5. In an embodiment, the final concentration of a LlF for the purpose of the process 100 according to the invention is between 5 ng/mL and 15 ng/mL, preferably between 7.5 ng/mL and 12.5 ng/mL, more preferably of 10 ng/mL. In an embodiment, the final concentration of an IL6 for the purpose of the process 100 according to the invention is between 5 ng/mL and 15 ng/mL, preferably between 7.5 ng/mL and 12.5 ng/mL, more preferably of 10ng/mL. In an embodiment, the final concentration of a SCF for the purpose of the process 100 according to the invention is between 0.5 ng/mL and 1.5 ng/mL, preferably between 0.75 ng/mL and 1.25 ng/mL, more preferably of 10 ng/mL. In an embodiment, the final concentration of an IGF-1 for the purpose of the process 100 according to the invention is between 1 ng/mL and 10 ng/mL preferably between 2.5 ng/mL and 7.5 ng/mL, more preferably of 10 ng/mL.

Another element of the culture medium for implementing the process 100 according to the invention is a combination of inhibitors that is able to impede the cells to initiate early differentiation steps.

Inventors have found that inhibiting MEK-, and Wnt-signalling is necessary but sufficient, together with the other culture conditions of the process 100 according to the invention as set forth herein to allow to impede stem cells to enter into early differentiation steps. Optionally, though inhibiting MEK-and Wnt-signalling is totally effective in isolating ESCs, it has been surprisingly found that further inhibiting PKC- signalling particularly improves reproducibility and success rate of the process according to the invention. Any compound or molecule that can lead to the inhibition of MEK- and/or Wnt-signalling pathways, and optionally to the inhibition of PKC-pathway, can be used. More particularly, said inhibitor compound or molecule can exert an inhibitory activity on a positive modulator of the targeted pathway and or on an element essential to the signal transduction in said pathways, which ultimately lead to the inhibition of signal transduction through this pathway. Alternatively, said inhibitor compound or molecule can exert an activating action on a negative modulator of the targeted pathway, which ultimately lead to the inhibition of signal transduction in the targeted pathway.

In an embodiment, an inhibitor leading to the inhibition of MEK-signalling pathway inhibits the mitogen-activated protein kinase enzymes MEK1 and/or MEK2. In another embodiment, an inhibitor leading to the inhibition of a Wnt-signalling pathway inhibits directly or indirectly either the canonical Wnt- pathway, the noncanonical planar cell polarity pathway, and the noncanonical Wnt/calcium pathway. More particularly, tankyrase inhibitors have been shown to stabilize axin and antagonize canonical Wnt-signalling. Accordingly, inhibitors of Wnt-signalling encompass tankyrase inhibitors.

In another embodiment, when present, the optional inhibitor leading to the inhibition of PKC-signalling pathway inhibits one or more members of protein kinase C family, for example selected from PKC α, PKC β, PKC γ, PKC δ, PKC ε, PKC η, PKC ζ or PKCµ.

Any means suitable for a use in cell culture conditions can be used in order to inhibit these signalling pathways. In an embodiment a siRNA can be used as inhibitor leading to the inhibition of any one of the MEK-, Wnt- or (optionally) PKC- signalling pathways while repressing expression proteins responsible of the activation and or signal transduction of, respectively, any of PKC-, MEK-, Wnt- or PKC- signalling pathways. siRNAs are known in the art as being small RNA molecules that interfere with the expression of genes having nucleotide sequences complementary to those of the siRNA. siRNA functions by breaking down mRNA after transcription. This results in the prevention of translation of the gene into protein. Candidate genes can be for example: MEK-1, Wnt1 or tankyrase, or a PKC. Customized siRNA are easily available for any gene on many provider platforms (see EUROFINS e. g. https://eurofinsgenomics.eu/en/ecom/tools/sirna-design/).

Small molecules also provide suitable inhibitors leading to the inhibition of any one of the MEK- or Wnt- or (optionally) PKC- pathways.

Examples of suitable small molecules to be used in the combination of inhibitors are listed in table 2 below.

**Table 2**

| **Name** | **CAS number / information** |
|---|---|
| **MEK-pathway inhibitors** | |
| binimetinib | 606143-89-9 |
| cobimetinib | 934660-93-2 |
| selumetinib | 606143-52-6 |
| trametinib | 871700-17-3 |
| PD035901 | 391210-10-9 |
| TAK-733 | 1035555-63-5 |
| PD-325901 | 391210-10-9 |
| CI-1040 | 212631-79-3 |
| PD98059 | 167869-21-8 |
| U0126 | 109511-58-2 |

| **Wnt-pathway inhibitors** | |
|---|---|
| adavivint | 1467093-03-3 |
| capmatinib | 1029712-80-8 |
| CCT251545 | 1661839-45-7 |
| FH 535 | 108409-83-2 |
| ginsenoside Rh4 | 174721-08-5 |
| ICG-001 | 847591-62-2 |
| iCRT14 | 677331-12-3 |
| IQ-1 | 331001-62-8 |
| isoquercitrin | 482-35-9 |
| IWP-2 | 686770-61-6 |
| IWP-4 | 686772-17-8 |
| IWR-1-endo | 1127442-82-3 |
| JW55 | 664993-53-7 |
| KY02111 | 1118807-13-8 |
| KY-05009 | 1228280-29-2 |
| KYA1797K | 1956356-56-1 |
| lanatoside C | 17575-22-3 |
| LF3 | 664969-54-4 |
| M2912 | 76362-12-4 |
| M435-1279 | 1359431-16-5 |
| MSAB | 173436-66-3 |
| NCB-0846 | 1792999-26-8 |
| PNU-74654 | 113906-27-7 |
| PRI-724 | 1422253-38-0 |
| prodigiosin | 82-89-3 |
| RCM-1 | 339163-65-4 |
| resibufogenin | 465-39-4 |
| salinomycin | 53003-10-4 |
| triptonide | 38647-11-9 |
| WIKI4 | 838818-26-1 |
| XAV-939 | 284028-89-3 |
| RXC004 | 1900754-56-4 |

| **PKC-pathway inhibitors** | |
|---|---|
| Gö6983 | 133053-19-7 |
| Gö6976 | 136194-77-9 |
| enzastaurin | 170364-57-5 |
| ruboxistaurin | 169939-94-0 |
| staurosporine | 62996-74-1 |
| GF 109203X (Bisindolylmaleimide I) | 133052-90-1 |
| ZIP | https://www.scbt.com/fr/p/pkc-zeta-pseudo-substrate-inhibitor-myristoylated |
| Ro 31-8220 | 125314-64-9 |
| Ro 32-0432 | 151342-35-7 |
| sotrastaurin | 425637-18-9 |
| rottlerin | 82-08-6 |
| K252a | 99533-80-9 |
| bisindolylmaleimide II | 133052-90-1 |
| calphostin C | 121263-19-2 |
| chelerythrine | 34316-15-9 |
| L-threo dihydrosphingosine | 15639-50-6 |
| melittin | 20449-79-0 |
| midostaurin | 120685-11-2 |

In an embodiment, the inhibitor of MEK-pathway to be used in the process 100 according to the invention is selected from binimetinib, cobimetinib, selumetinib, trametinib, CI-1040, PD035901, TAK-733, PD-325901, PD98059 or U0126, or a mix thereof.

In an embodiment, the inhibitor of Wnt-pathway to be used in the process 100 according to the invention is selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004, or a mix thereof.

In a particular embodiment, to implement the process 100 of the invention, the culture medium comprises at least one inhibitor selected from binimetinib, cobimetinib, selumetinib, trametinib, CI-1040, PD035901, TAK-733, PD-325901, PD98059 or U0126 and at least one inhibitor selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004.

In a particular embodiment, to implement the process 100 of the invention, the culture medium comprises at least one inhibitor selected from binimetinib, cobimetinib, selumetinib, trametinib, CI-1040, PD035901, TAK-733, PD-325901, PD98059 or U0126, at least one inhibitor selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 and at least one inhibitor selected from Gö6983, Gö6976, enzastaurin, ruboxistaurin, staurosporine, GF 109203X (Bisindolylmaleimide I), ZIP, Ro 31-8220, Ro 32-0432, sotrastaurin, rottierin, K252a, bisindolylmaleimide II, calphostin C, chelerythrine , L-threo dihydrosphingosine, melittin, or midostaurin.

In an embodiment, the inhibitor of PKC-pathway, when used in the process 100 according to the invention, is selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin, or a mix thereof.

In an embodiment, in the process 100 of the invention, the culture medium comprises PD-325901 as an inhibitor leading to the inhibition of MEK-signalling. In a more particular embodiment, in the process 100 of the invention the culture medium comprises PD-325901 as an inhibitor leading to the inhibition of MEK-signalling and at least one inhibitor leading to the inhibition of Wnt-signalling selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 or a combination thereof. In another particular embodiment, in the process 100 of the invention the culture medium comprises PD-325901 as an inhibitor leading to the inhibition of MEK-signalling and at least one inhibitor leading to the inhibition of PKC-signalling- selected from Gö6983, Gö6976, enzastaurin, ruboxistaurin, staurosporine, GF 109203X (Bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin or a combination thereof.

In a more particular embodiment, in the process 100 of the invention the culture medium comprises PD98059 as an inhibitor leading to the inhibition of MEK-signalling and at least one inhibitor leading to the inhibition of Wnt-signalling selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 or a combination thereof. In another particular embodiment, in the process 100 of the invention the culture medium comprises PD98059 as an inhibitor leading to the inhibition of MEK-signalling and at least one inhibitor leading to the inhibition of PKC-signalling- selected from Gö6983, Gö6976, enzastaurin, ruboxistaurin, staurosporine, GF 109203X (Bisindolylmaleimide I), ZIP, Ro 31-8220, Ro 32-0432, sotrastaurin, Rottlerin, K252a, Bisindolylmaleimide II, calphostin C, chelerythrine, L-threo dihydrosphingosine, melittin, midostaurin or a combination thereof.

In a more particular embodiment, in the process 100 of the invention the culture medium comprises U0126 as an inhibitor leading to the inhibition of MEK-signalling and at least one inhibitor leading to the inhibition of Wnt-signalling selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 or a combination thereof. In another particular embodiment, in the process 100 of the invention the culture medium comprises U0126 as an inhibitor leading to the inhibition of MEK-signalling and at least one inhibitor leading to the inhibition of PKC-signalling- selected from Gö6983, Gö6976, enzastaurin, ruboxistaurin, staurosporine, GF 109203X (Bisindolylmaleimide I), ZIP, Ro 31-8220, Ro 32-0432, sotrastaurin, Rottlerin, K252a, Bisindolylmaleimide II, calphostin C, chelerythrine, L-threo dihydrosphingosine, melittin, midostaurin or a combination thereof.

In a more particular embodiment, in the process 100 of the invention the culture medium comprises binimetinib as an inhibitor leading to the inhibition of MEK-signalling and at least one inhibitor leading to the inhibition of Wnt-signalling selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 or a combination thereof. In another particular embodiment, in the process 100 of the invention the culture medium comprises binimetinib as an inhibitor leading to the inhibition of MEK-signalling and at least one inhibitor leading to the inhibition of PKC-signalling- selected from Gö6983, Gö6976, enzastaurin, ruboxistaurin, staurosporine, GF 109203X (Bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin or a combination thereof.

In a particular embodiment, in the process 100 of the invention the culture medium comprises a tankyrase inhibitor as an inhibitor leading to the inhibition of Wnt- signalling. In a more particular embodiment, in the process 100 of the invention the culture medium comprises a tankyrase inhibitor as an inhibitor leading to the inhibition of Wnt- and at least one inhibitor leading to the inhibition of MEK-signalling selected from binimetinib, cobimetinib, selumetinib, trametinib, CI-1040, PD035901, TAK-733, PD-325901, PD98059 or U0126 or a combination thereof. In another particular embodiment, in the process 100 of the invention the culture medium comprises a tankyrase inhibitor as an inhibitor leading to the inhibition of Wnt- signalling and at least one inhibitor leading to the inhibition of PKC-signalling selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin or a combination thereof.

In a particular embodiment, in the process 100 of the invention the culture medium comprises XAV-939 as an inhibitor leading to the inhibition of Wnt- signalling. In a more particular embodiment, in the process 100 of the invention the culture medium comprises XAV-939 as an inhibitor leading to the inhibition of Wnt- signalling and at least one inhibitor leading to the inhibition of MEK-signalling selected from binimetinib, cobimetinib, selumetinib, trametinib, CI-1040, PD035901, TAK-733, PD-325901, PD98059 or U0126 or a combination thereof.. In another particular embodiment, in the process 100 of the invention the culture medium comprises XAV-939 as an inhibitor leading to the inhibition of Wnt- signalling and at least one inhibitor leading to the inhibition of PKC-signalling selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin or a combination thereof.

In a more particular embodiment, in the process 100 of the invention the culture medium comprises IWP-2 as an inhibitor leading to the inhibition of Wnt- signalling and at least one inhibitor leading to the inhibition of MEK-signalling selected from binimetinib, cobimetinib, selumetinib, trametinib, CI-1040, PD035901, TAK-733, PD-325901, PD98059 or U0126 or a combination thereof. In another particular embodiment, in the process 100 of the invention the culture medium comprises IWP-2 as an inhibitor leading to the inhibition of Wnt- signalling and at least one inhibitor leading to the inhibition of PKC-signalling selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin or a combination thereof.

In a more particular embodiment, in the process 100 of the invention the culture medium comprises adavivint as an inhibitor leading to the inhibition of Wnt- signalling and at least one inhibitor leading to the inhibition of MEK-signalling selected from binimetinib, cobimetinib, selumetinib, trametinib, CI-1040, PD035901, TAK-733, PD-325901, PD98059 or U0126 or a combination thereof. In another particular embodiment, in the process 100 of the invention the culture medium comprises adavivint as an inhibitor leading to the inhibition of Wnt-signalling and at least one inhibitor leading to the inhibition of PKC-signalling selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin or a combination thereof.

In a more particular embodiment, in the process 100 of the invention the culture medium comprises capmatinib as an inhibitor leading to the inhibition of Wnt- signalling and at least one inhibitor leading to the inhibition of MEK-signalling selected from binimetinib, cobimetinib, selumetinib, trametinib, CI-1040, PD035901, TAK-733, PD-325901, PD98059 or U0126 or a combination thereof. In another particular embodiment, in the process 100 of the invention the culture medium comprises capmatinib as an inhibitor leading to the inhibition of Wnt-signalling and at least one inhibitor leading to the inhibition of PKC-signalling selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin or a combination thereof.

In a further particular embodiment, in the process 100 of the invention the culture medium comprises Gö6983 as an inhibitor leading to the inhibition of PKC- signalling. In a more particular embodiment, in the process 100 of the invention the culture medium comprises Gö6983 as an inhibitor leading to the inhibition of PKC- signalling and at least one inhibitor leading to the inhibition of MEK-signalling selected from binimetinib, cobimetinib, selumetinib, trametinib, CI-1040, PD035901, TAK-733, PD-325901, PD98059 or U0126, or a combination thereof. In another particular embodiment, in the process 100 of the invention the culture medium comprises Gö6983 as an inhibitor leading to the inhibition of PKC- signalling and at least one inhibitor of Wnt-pathway selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 or a combination thereof. In a more particular embodiment, in the process 100 of the invention the culture medium comprises Gö6976 as an inhibitor leading to the inhibition of PKC- signalling and at least one inhibitor leading to the inhibition of MEK-signalling selected from binimetinib, cobimetinib, selumetinib, trametinib, CI-1040, PD035901, TAK-733, PD-325901, PD98059 or U0126, or a combination thereof. In another particular embodiment, in the process 100 of the invention the culture medium comprises Gö6976 as an inhibitor leading to the inhibition of PKC- signalling and at least one inhibitor of Wnt-pathway selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 or a combination thereof. In a more particular embodiment, in the process 100 of the invention the culture medium comprises enzastaurin as an inhibitor leading to the inhibition of PKC- signalling and at least one inhibitor leading to the inhibition of MEK-signalling selected from binimetinib, cobimetinib, selumetinib, trametinib, CI-1040, PD035901, TAK-733, PD-325901, PD98059 or U0126, or a combination thereof. In another particular embodiment, in the process 100 of the invention the culture medium comprises enzastaurin as an inhibitor leading to the inhibition of PKC- signalling and at least one inhibitor of Wnt-pathway selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 or a combination thereof. In a more particular embodiment, in the process 100 of the invention the culture medium comprises ruboxistaurin as an inhibitor leading to the inhibition of PKC- signalling and at least one inhibitor leading to the inhibition of MEK-signalling selected from binimetinib, cobimetinib, selumetinib, trametinib, CI-1040, PD035901, TAK-733, PD-325901, PD98059 or U0126, or a combination thereof. In another particular embodiment, in the process 100 of the invention the culture medium comprises ruboxistaurin as an inhibitor leading to the inhibition of PKC- signalling and at least one inhibitor of Wnt-pathway selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004 or a combination thereof.

In a particular embodiment the culture medium to be used in the process 100 according to the invention comprises at least one of the combination of inhibitors selected from :
- adavivint and Gö6976,
- binimetinib and Gö6983,
- capmatinib and IWP-2,
- enzastaurin and PD-325901,
- Gö6976 and PD-325901,
- Gö6983 and PD98059,
- IWP-2 and ruboxistaurin,
- PD-325901 and U0126,
- PD98059 and adavivint,
- ruboxistaurin and binimetinib,
- U0126 and capmatinib,
- XAV-939 and enzastaurin,
- XAV-939 and PD98059,
- XAV-939 and Gö6983,
- Gö6976 and capmatinib and IWP-2,
- Gö6983 and PD98059 and adavivint,
- Gö6983 and PD-325901 and Xav-939,
- ruboxistaurin and PD-325901 and binimetinib, or
- enzastaurin and XAV-939 and U0126.

In a particular embodiment, each small molecule acting as an inhibitor in the combination of inhibitors as described above is present at a final concentration in the cell culture medium of between 0.05 µM to 4 µM, preferably between 0.1 µM to 2 µM, preferably 0.5 µM to 1 µM.

In a more particular embodiment, in the process 100 of the invention the culture medium comprises :
- at least one MEK-signalling inhibitor at a final concentration in the cell culture medium of between 0.05 µM to 2 µM, preferably between 0.5 µM to 1 µM, and/or
- at least one Wnt- signalling inhibitor at a final concentration in the cell culture medium of between 0.5 µM to 4 µM, preferably between 0.1 µM to 2 µM.

In a more particular embodiment, in the process 100 of the invention the culture medium comprises :
- at least one MEK-signalling inhibitor at a final concentration in the cell culture medium of between 0.05 µM to 2 µM, preferably between 0.5 µM to 1 µM,
- at least one Wnt- signalling inhibitor at a final concentration in the cell culture medium of between 0.5 µM to 4 µM, preferably between 0.1 µM to 2 µM,
- at least one PKC inhibitor at a final concentration in the cell culture medium of between 0.5 µM to 4 µM, preferably between 0.1 µM to 2 µM.

The other important feature to implement the process 100 of the invention and successfully isolate avian ESCs, is the culture medium. Culture conditions as determined by the inventors allow the use of serum free medium. In other words, there is no need to complement the medium with a serum of animal origin. More preferably, the animal serum substitute used in the process 100 of the invention contains few, if any, components of animal origin. When they contain components of animal origin, these are purified and well defined compounds (transferrin, BSA etc...) in known quantities, ensuring no negative impact on reproducibility. This is of particular interest as it allows to avoid unpredictability in relation with unwanted and uncontrollable variations that can occur when using products of animal origin like foetal bovine serum (FBS). In other words, this feature, besides allowing the good isolation of avian ESCs, contribute to the high reproducibility rate of the process 100 of the invention. Also, avoiding the use of animal derived products fulfils a growing concern, for instance in terms of animal welfare for FBS or safety concerns for human consumption, and makes a particular sense in case said ESCs are considered to be used for a pharmaceutical purpose or making cultivated meat, or any edible food product, or even lab-grown leather.

Such animal-serum substitutes are commercially available. Media that can be used in the process 100 of the invention are, e.g.:
- KNOCKOUT^{™}SR (Gibco),
- Serum Replacement Basic (Genaxxon Bioscience),
- Serum Replacement 3 (Sigma),
- B27 Supplement ^{™} serum-free (ThermoFisher), or
- BIT 9500 Serum Substitute (StemCell).

These substitutes are free of animal serum and contain no or few compounds of animal origin. These compounds are clearly identified and used at a defined concentration.

As used herein, "basal culture medium" meant a culture medium which allows, by itself, at least cell survival, and even better, cell growth. Examples of suitable basal culture media are BME (basal Eagle Medium), MEM (minimum Eagle Medium), 20 medium 199, DMEM (Dulbecco's modified Eagle Medium), GMEM (Glasgow modified Eagle, medium), DMEM F-12, Ham-F12, Ham-F10, Iscove's Modified Dulbecco's medium, MacCoy's 5A medium, RPMI 1640, GTM3, or a combination thereof. The basal medium comprises inorganic salts (e.g. CaCl₂, KCI, NaCl, NaHCOs, NaH₂PO₄, MgSO₄, etc.), amino-acids, vitamins (thiamine, riboflavin, folic acid, D-Ca panthothenate,etc. ) and other components such as glucose, 2 mercapto-ethanol, sodium pyruvate. Preferably the basal medium is a synthetic medium.

Composition of DMEM / F-12 as an exemplary culture medium suitable for the invention is provided in table 3 below.

**Table 3**

| **Components** | **Concentration (mM)** |
|---|---|
| **Amino Acids** | |
| Glycine | 0.250 |
| L-Alanine | 0.050 |
| L-Arginine hydrochloride | 0.699 |
| L-Asparagine-H₂O | 0.050 |
| L-Aspartic acid | 0.050 |
| L-Cysteine hydrochloride-H₂O | 0.100 |
| L-Cystine 2HCl | 0.100 |
| L-Glutamic Acid | 0.050 |
| L-Glutamine | 2.500 |
| L-Histidine hydrochloride-H₂O | 0.150 |
| L-Isoleucine | 0.416 |
| L-Leucine | 0.451 |
| L-Lysine hydrochloride | 0.499 |
| L-Methionine | 0.116 |
| L-Phenylalanine | 0.215 |
| L-Proline | 0.150 |
| L-Serine | 0.250 |
| L-Threonine | 0.449 |
| L-Tryptophan | 0.044 |
| L-Tyrosine disodium salt dihydrate | 0.214 |
| L-Valine | 0.452 |

| **Vitamins** | |
|---|---|
| Biotin | 1.43E-05 |
| Choline chloride | 0.064 |
| D-Calcium pantothenate | 0.005 |
| Folic Acid | 0.006 |
| Niacinamide | 0.017 |
| Pyridoxine hydrochloride | 0.010 |
| Riboflavin | 5.82E+02 |
| Thiamine hydrochloride | 0.006 |
| Vitamin B12 | 5.01E-04 |
| i-Inositol | 0.070 |

| **Inorganic Salts** | |
|---|---|
| Calcium Chloride (CaCl2) (anhyd.) | 1.050 |
| Cupric sulfate (CuSO4-5H₂O) | 5.20E-06 |
| Ferric Nitrate (Fe(NO₃)3"9H₂O) | 1.24E+03 |
| Ferric sulfate (FeSO₄-7H₂O) | 0.002 |
| Magnesium Chloride (anhydrous) | 0.301 |
| Magnesium Sulfate (MgSO₄) (anhyd.) | 0.407 |
| Potassium Chloride (KCI) | 4.157 |
| Sodium Bicarbonate (NaHCOs) | 29.024 |
| Sodium Chloride (NaCl) | 120.612 |
| Sodium Phosphate dibasic (Na₂HPO₄) anhydrous | 0.500 |
| Sodium Phosphate monobasic (NaH₂PO₄-H₂O) | 0.453 |
| Zinc sulfate (ZnSO4-7H₂O) | 0.0015 |

| **Other components** | |
|---|---|
| D-Glucose (Dextrose) | 17.506 |
| Hypoxanthine Na | 0.015 |
| Linoleic Acid | 1.50E-04 |
| Lipoic Acid | 5.10E-04 |
| Phenol Red | 0.022 |
| Putrescine 2HCl | 5.03E-04 |
| Sodium Pyruvate | 0.500 |
| Thymidine | 0.002 |

The basal culture medium is typically supplemented with non-essential amino acids, sodium pyruvate, L-glutamine, 2 mercaptoethanol and a mix of penicillin and streptomycin.

Typically basal culture medium of the invention may be complemented with additives selected in the list below :
- 0.1 mM to 5 mM L-glutamine, preferably between 2 to 3 mM;
- 0.05 mM to 2 mM sodium pyruvate, preferably between 0.5 mM to 1.5 mM sodium pyruvate;
- 0.05 mM to 2 mM 2 mercapto-ethanol, preferably 0.1 mM to 1 mM ;
- 0.5 % to 2 % non-essential amino acids.

### Step c) of seeding the suspension of embryonic cells 103 obtained in step b) on a layer of feeder cells,

In the process 100 of the invention, cells of at least one dissected embryo obtained from the previous steps are seeded 103 on a layer of feeder cells which line the bottom of a culture well. Alternatively, but less preferably, the feeder cells can be substituted with extra-cellular matrix that can also contain bound growth factors which coat the petri dish surface. In an embodiment, feeder cells are mitotically inactivated using standard techniques (irradiation or with a double-stranded DNA alkylating agent).

Typically, STO fibroblasts are preferred and can be of any mammalian, (e.g. rat, ungulate, bovine, porcine species); or avian species.

In a particular embodiment, feeder cells are proliferative murine STO cells. In an even more particular embodiment, feeder cells are STO CRL-1503^{™} (American Type Culture Collection (ATCC)).

In another embodiment, feeder cells are grown in the culture in a mono-layer until about 80% of confluency, about 90% of confluency, and even about 100% of confluency is reached.

In another embodiment, the feeder cells are replaced by an organic extracellular matrix or feeder cell-conditioned medium. Non-limiting examples of an organic matrix are Matrigel, Vitronectin, or any organic matrix known by the person skilled in the art. In a yet further aspect, the feeder cells are replaced by an organic matrix supplemented with Activin-A, e.g. with 10 ng/mL of Activin-A.

### Step d) of culturing 104 the seeded embryonic cells for at least one passage.

A daily media change can be performed; especially the first days of culture, when a lot of cell debris and lipids can be present.

ESCs are supposed to be passed at confluence time. A passage is defined as above.

Typically, 1 to 20 passages or even more can be performed.

ESCs isolated through the process 100 of the invention can be then stored by cryopreservation or subjected to progressive weaning in the above-mentioned growth factors and/or feeder cells and/or inhibitors, in order to obtain a stable cell line of ESCs.

Cells are cultured in a controlled atmosphere, at a temperature which can be between 35°C to 39°C, preferably 37°C, under a concentration of CO₂ of above 5% and below 10%, preferably between 6 % and 9%, even preferably 7.5%.

In a particular embodiment, the process 100 of the invention can comprise a further step of controlling the pluripotency of the cells 105 obtained at step d). Such controlling step can be performed by measuring the expression level of a set of pluripotency markers. The expression level can be measured e.g. by RT-qPCR or immunological methods (FACS, immunofluorescence). These pluripotency markers are for example selected from one or more of OCT4, TERT, DNMT3B, NANOG, CXCL5, DNMT3B, HESX1, IDO1, LCK, POU5F1, SOX2, TRIM22, EMA1, SSEA1. Preferably, pluripotency is evaluated by determining pluripotency expression profile of the cells by measuring expression of at least 4 of pluripotency markers as, e.g., those listed above. Cells are considered as having kept their pluripotency property when their pluripotency expression profile is at least 60%, at least 70%, at least 80%, at least 90% and even 100% identity with control pluripotent cells. Some bioinformatics assay are commercially available to evaluate pluripotency of cells (https://www.thermofisher.com/fr/fr/home/life-science/stem-cell-research/induced-pluripotent-stem-cells/pluripotent-stem-cell-detection.html). In an embodiment, cells are considered as having kept their pluripotency feature when at least 3 out of 4 of pluripotency markers are maintained at a level higher than in differentiated cells. Pluripotency expression profile evaluation can be associated to telomerase expression assays or any pluripotency assay known by the person skilled in the art.

Accordingly, ESCs can be controlled, e.g., at each passage, for their pluripotency as exposed above. In a particular embodiment, a population fulfilling the above criteria after a fifth passage, or even a further passage, is considered as a valuable ESC line. Obviously, the more the ESCs have been passed while conserving their pluripotency, the more stable the ESCs line is supposed to be. Accordingly, in a preferred embodiment of the process 100 according to the invention, the ESCs have been subjected to at least 5 passages, 6, 7, 8, 9, 10, 11, 12 13, 14, 15, 16, 17, 18, 19, even 20 passages while keeping their pluripotency.

A second object of the invention is a process of obtaining a continuous diploid cell line 200 from the ESCs isolated as above.

### Process of obtaining a continuous diploid cell line 200.

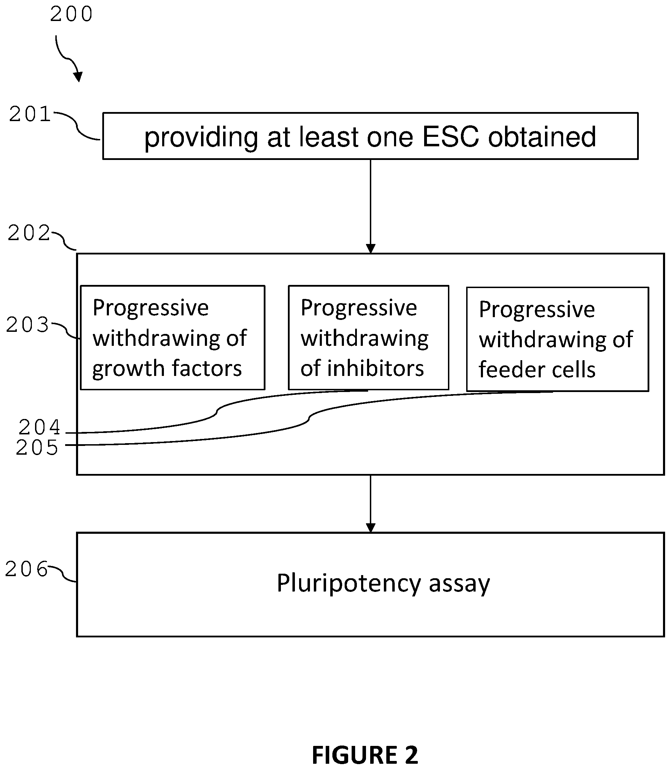

As illustrated figure 2, a process of obtaining a continuous diploid cell line 200 subjected to the second object of the invention comprises all the steps of the process 100 of isolating avian ESCs as exposed above in any of its embodiment (step of providing at least one ESC obtained 201) with a further step of a weaning process 202 that comprises a progressive withdrawal (for example a decrease of 25% every 2 or 3 passages) of any of the growth factors 203, the inhibitors 204 (for example a decrease of 25% every 2 or 3 passages) and/or the feeders cells 205 (for example a decrease of 25% every 2 or 3 passages), in the culture medium of the ESCs in the same way as the one disclosed in WO2008/129058, *mutatis mutandis,* in order to wean the cells from their dependency to the said growth factors, inhibitors and feeders cells. Said withdrawal can be performed simultaneously, successively, or separately.

Sequences of withdrawals 203, 204, 205 can be, but are not limited to, for example:
- feeder cells / inhibitors / growth factors;
- feeder cells / growth factors / inhibitors;
- inhibitors / growth factors / feeder cells;
- inhibitors / feeder cells / growth factors;
- growth factors / inhibitors / feeder cells; or
- growth factors / feeder cells / inhibitors.

The cells obtained at the end of the step of the weaning process 202 are pluripotent cells which can divide indefinitely without growth factors. The pluripotency of cells can be assayed in a further step 206 as explained previously for the further step 105 of process 100.

The inventors have been able to determine the specific compounds and products that can be used in order to isolate avian ESCs and derive them from a continuous pluripotent cell line. Accordingly, a third object of the invention is a kit of parts that is useful in implementing the processes that are the objects of the invention.

### Kit of parts adapted for reconstituting a cell culture medium suitable to implement a process according to the invention.

A kit useful for implementing processes 100, 200 of the invention and reconstituting a cell culture medium can comprise:
- a set of growth factors comprising one or more of IL6, leukemia inhibitory factor LIF, IGF-1, or SCF, or any ortholog thereof;
- at least one inhibitor leading to the inhibition of MEK-, Wnt-, and optionally PKC- signalling as defined above.

Also in a more particular embodiment, the invention relates to a kit further comprising an animal serum substitute.

In a particular embodiment, in the kit according to the invention comprises:
a. at least one inhibitor of MEK signalling selected from i) a small molecule selected from binimetinib, cobimetinib, selumetinib, trametinib, CI-1040, PD035901, TAK-733, PD-325901, or a mix thereof, or ii) a MEK-1 siRNA,
b. at least one inhibitor of Wnt signalling is selected from i) a small molecule selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, RXC004, or a mix thereof ii) a Wnt-1 siRNA, and
c. at least inhibitor of PKC signalling selected from i) a small molecule selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, midostaurin, or a mix thereof, or a mix thereof or ii) a PKC-alpha siRNA.

In a particular embodiment, in the kit according to the invention, at least one inhibitor and the growth factors are conditioned so as to allow to reach their final concentration in the reconstituted culture medium used in the process for isolating the ESCs 100 of the invention.

In a further particular embodiment, the kit according to the invention can comprise material useful to assay the pluripotency of the cell resulting from the implementation of the process 100, 200. Accordingly, in this embodiment, the kit can comprise sets of oligonucleotides allowing the determination of pluripotency expression profile as mentioned above, for example at least a set of oligonucleotides suitable to measure the expression level of at least one or more of OCT4, TERT, DNMT3B, NANOG, CXCL5, DNMT3B, HESX1, IDO1, LCK, POU5F1, SOX2, or TRIM22 pluripotency markers.

The following examples are given for purposes of illustration and not by way of limitation.

### EXAMPLES

### I. Material and method

### a. Material

### Duck Eggs

Less than 3 days old certified Pekin duck eggs (Orvia, France) :
- controlled for avian influenza,
- no residues from veterinary medicines, pesticides, contaminants,
- no *Salmonella,*
- no in-ovo vaccination.

The eggs have been stored between 18-21°C following collection at oviposition and stored, if needed to block embryo development. Eggs are used either on the day of collection or the following days after delivery. Accordingly, extracted embryos were less than 3 days old.

### Chicken Eggs

Less than 3 days old certified chicken eggs (Orvia, France) :
- controlled for avian influenza,
- no residues from veterinary medicines, pesticides, contaminants,
- no *Salmonella,*
- no in-ovo vaccination,

The eggs have been stored between 18-21°C following collection at oviposition and stored, if needed to block embryo development. Eggs are used either on the day of collection or the following days after delivery. Accordingly, extracted embryos were less than 3 days old.

### Media and culture

DMEM/F12-KO (Thermofisher, #12660-012)
BIT9500 (Stemcell technologies, # 09500)
Non-essential amino acid (Thermofisher, #11140-050)
Sodium pyruvate (Thermofisher, #11530-396)
L-glutamine (Thermofisher, #2503123)
2-mercaptoethanol (Thermofisher, #31350-010)
Penicillin/Streptomycin (Thermofisher, #11548876)
Feeder cells : STO CRL-1503^{™}

### Antibodies

Primary antibodies : anti SSEA1: ThermoFisher (Cat #MA1-022) or Abcam (ref: ab16285) or Santa Cruz (ref sc-21702) ; anti EMA1: Abcam (ref: ab109110).
Secondary antibody : Anti-Mouse IgM (Alexa-488).

### Growth factors

Recombinant growth factors have been produced internally using common biology molecular tools, based upon sequences retrieved in the Uniprot database and listed in table 1. Human and chicken growth factors are otherwise commercially available. Growth factors have been tested at concentrations ranging from 1 ng/mL to 10 ng/mL.

### Inhibitors

Tested inhibitors are used at final concentrations ranging from 1 to 2 µM. They are commercially available and can be retrieved using either CAS number or their name as listed in table 2.

### b. Isolation of ESCs from duck embryo or chicken embryo

Five eggs are first washed at room temperature with a tissue soaked in ethanol, they are then carefully broken so as to keep the yolk intact and the white is gently separated from the yolk and the yolk is placed in a14 cm Petri-dish with the embryo on the top.

The excess of white on the surface of the embryo using Whatman^{®} papers. A Whatman^{®} paper with the hole surrounding each of the embryo is placed on the embryo, and 3-4 small incisions outside the paper disk are made with scissors (let in a 70% ethanol solution) and the paper carrying each of the embryo is isolated from the yolk with forceps (also let in a 70% ethanol solution) and placed on the other side (embryo on the surface) in a new 14cm dish previously filled with cold PBS.

Each embryo is then cleaned and isolated with 0.5mL PBS sprayed and aspirated with a 30G needle on a 1mL syringe. Each embryo is then aspirated with a 1000µL pipette tip.

The epiblast region of the embryo is removed to collect only the center of the embryos and put in a 15mL falcon containing 1mL of PBS at room temperature. Dissected embryos are then centrifuged 5 minutes at 300g at room temperature and resuspended in the tested media.

STO medium is removed from the cultured plates comprising feeder cells which are then rinsed once with PBS each wells, and the cells are then seeded and incubated in a static at 37°C, 7.5% CO₂. 3 wells are seeded with dissected embryos.

The day after the isolation, the medium is changed for 1.5 mL of medium at 37°C. 1 or 2 rinses with room temperature PBS can be made if too much lipids and cell debris are present.

A daily media change and a first passage is done as soon as an ESCs population shows up. After that, cells have to be passed at confluence time.

Pluripotency can be tested at each passage.

### RTqPCR

RNA is extracted from a cell pellet gathered from cell cultures 3 wells of a 12 wells plate, using Monarch^{®} Total RNA Miniprep Kit (NEB).

Reverse transcription of RNA extracted from the cells was performed using "High-Capacity cDNA Reverse Transcription Kit" (ThermoFicher scientific), according to the instructions of the supplier.

For qPCR, oligonucleotides for OCT4, TERT, DNMT3B, NANOG, CXCL5, DNMT3B, HESX1, IDO1, LCK, POU5F1, SOX2 and TRIM22 were designed using the EUROFIN primer design tool available under : https://eurofinsgenomics.eu/en/dna-rna-oligonucleotides/oligo-tools/primer-design-tools/?gclid=Cj0KCOiAgqGrBhDtARIsAM5s0_IEU30NpeE072pgv1bwpxYKJztJfFPqrj7jWL ksG0vXf8GrV8rGsYMaAgS8EALw_wcB, and the sequence of the gene obtained from NCBI database.

### IHC assay for EMA1 and SSEA-1

Cells are cultured into a 12 wells plate containing coated glass coverslides containing feeder cells until they reach at least 80% confluence.

Wells are then rinsed with room temperature PBS and cells are fixed by adding 4% paraformaldehyde solution (PFA) (0.5 mL), for 15 minutes at room temperature.

PFA is then removed and wells are rinsed three times with PBS at room temperature for 5 minutes each under very gentle rocking.

### Staining

PBS is removed and cells are incubated for 20 min in blocking solution (BS) - DPBS, 10% goat serum, 1 % of bovine serum albumin (BSA) (0.5 mL), under gentle rocking.

BS is removed and primary antibody (anti SSEA1 or anti EMA 1) solution is added at the dilution prescribed by the supplier for an overnight incubation à 4°C under gentle rocking (50-60 rpm).

Primary antibody solution is removed, and wells are rinsed 3 times with DPBS-Tween 20 (0.05%) for 6-10 minutes under gentle rocking (50- 60 rpm) before incubation with the suitable antibody solution and DAPI for 1h at room temperature, if possible under small agitation (50-60 rpm).

Wells are rinsed 3 times, at room temperature, with DPBS-Tween 20 (0.05%) for 6-10min minutes under gentle rocking (50-60 rpm) rm.

### Mounting and Imaging

The slide with cells is laid face-down on 13-15 µL of mounting medium (ProLong Gold antifade reagent - Invitrogen P36930) to a glass slide.

Slides are ready to be analysed under fluorescence microscopy.

### II. Results

Table 4 below gather some of the assays that are currently made

**Table 4**

| **assay** | 1 | 2 | 3 | 4 | 5 | 6 | 7 | 8 |
|---|---|---|---|---|---|---|---|---|
| **embryo** | duck | duck | duck | duck | duck | chicken | chicken | chicken |
| **growth factors** | Chicken LlF | Chicken LlF | Chicken LlF | Chicken LlF | Chicken LlF | Human LlF | Human LlF | Chicken CNTF |
| | Chicken IL6 | Chicken IL6 | Chicken IL6 | Chicken IL6 | Chicken IL6 | Human IL6 | Human IL6-R | Chicken IL6 |
| | Chicken SCF | Chicken SCF | Chicken SCF | Chicken SCF | Chicken FGF2 | Human SCF | Human FGF2 | Chicken FGF2 |
| | Chicken IGF1 | Chicken IGF1 | Chicken IGF1 | Chicken IGF1 | Chicken IGF1 | Human IGF1 | Human IGF1 | Chicken IGF1 |
| | | Chicken FGF2 | | | | | | Chicken SCF |
| **maintenance of an undifferentiated state** | - | - | Wnt inhibitor | Wnt inhibitor MEK inhibitor | Wnt inhibitor | Wnt inhibitor | Wnt inhibitor | - |
| | | | MEK inhibitor | | MEK inhibitor | MEK inhibitor | MEK inhibitor | |
| | | | PKC inibitor | | PKC inibitor | PKC inibitor | PKC inibitor | |
| plutipotency at passage 5* | no | no | yes | yes | yes | yes | yes | no |
| plutipotency at passage 8* | no | no | yes | yes | yes | yes | yes | no |
| plutipotency at passage 20** | no | no | yes | yes | yes | yes | yes | no |

| | | | | | | | | |
|---|---|---|---|---|---|---|---|---|
| * evaluated by RT-qPCR **evaluated by IHC | | | | | | | | |

As shown in table 4, the presence in the culture medium of a combination of growth factors either from chicken or human targeting the pathways identified by the inventors allows the isolation of ESCs from embryos (assays 3-7).

Further, the presence of at least a MEK-signalling inhibitor and of at least Wnt-signalling inhibitor is necessary (assay 1,2 versus assays 3-7) to isolate and maintain ESCs.

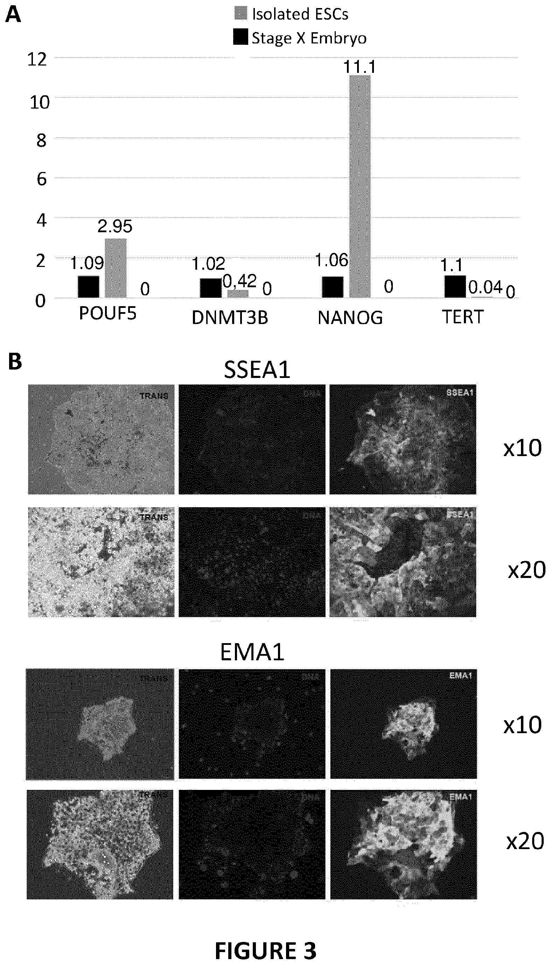

As illustrated for assay 3 (Figure 3) as well as for assays (3-7) the ESCs keep their pluripotency phenotype over the passages. Under these conditions, the use of the combination of growth factors as disclosed in the state of the art is inoperant (assay 8).

Of note 100% of the ESC isolation processes according to conditions of assay 3-7 succeeded in obtaining pluripotent ESC line (n=4). Illustrative data of pluripotency assay obtained for one ESC line obtained using the process of the invention is given on figure 3A. Pluripotency is confirmed and results from RT-qPCR confirm pluripotency is maintained up to 20 passages.

ESCs isolation is successful for any of the tested combination of a MEK-, Wnt-, and optionally PKC signalling pathways inhibitors, particularly the following combinations :
- adavivint and Gö6976,
- binimetinib and Gö6983,
- capmatinib and IWP-2,
- enzastaurin and PD-325901,
- Gö6976 and PD-325901,
- Gö6983 and PD98059,
- IWP-2 and ruboxistaurin,
- PD-325901 and U0126,
- PD98059 and adavivint,
- ruboxistaurin and binimetinib,
- U0126 and capmatinib,
- XAV-939 and enzastaurin,
- XAV-939 and PD98059, or
- XAV-939 and Gö6983,
- Gö6976 and capmatinib and IWP-2,
- Gö6983 and PD98059 and adavivint,
- Gö6983 and PD-325901 and Xav-939,
- ruboxistaurin and PD-325901 and binimetinib, and
- enzastaurin and XAV-939 and U0126.

### BIBLIOGRAPHY

Eyal-Giladi H, Kochav S. From cleavage to primitive streak formation: a complementary normal table and a new look at the first stages of the development of the chick. I. General morphology. Dev Biol. 1976 Apr;49(2):321-37.
Sellier N., Brillard J.-P., Dupuy V., Bakst M.R.. Comparative Staging of Embryo Development in Chicken, Turkey, Duck, Goose, Guinea Fowl, and Japanese Quail Assessed from Five Hours After Fertilization Through Seventy-Two Hours of Incubation, Journal of Applied Poultry Research, Volume 15, Issue 2, 2006, pages 219-228.
UniProt Consortium. UniProt: the Universal Protein Knowledgebase in 2023. Nucleic Acids Res. 2023 Jan 6;51(D1):D523-D531

## Claims

1. A process for isolating embryonic stem cells (100) from at least one avian embryo comprising the steps of :
a. isolating at least one embryo (101) at a developmental stage around oviposition,
b. suspending embryonic cells (102) obtained by dissociating embryo(s) of step a) in an animal serum free basal culture medium supplemented with :
- a mix of growth factors which modulates at least the following pathways : JAK/STAT, PI3K/AKT, SHP2/MAPK, PLC-gamma, MAPK, PI3K/AKT/MTOR, RAS/RAF, RHOA/ROCK
- at least one inhibitor leading to the inhibition of MEK- signalling,
- at least one inhibitor leading to the inhibition of Wnt-signalling, and
- a substitute to animal serum,
c. seeding the suspension of embryonic cells (103) obtained in step b) on a layer of feeder cells,
d. culturing (104) the embryonic cells for at least one passage.

2. The process for isolating embryonic stem cells (100) according to claim 1 wherein the mix of growth factors comprises at least one ortholog of the following growth factors : interleukin 6 (IL6), Leukemia inhibitory factor (LIF), Insulin Growth factor 1 (IGF-1), and Stem Cell Factor (SCF).

3. The process for isolating embryonic stem cells (100) according to claim 2, wherein :
- IL6 is selected from avian IL6 or mammalian IL6, or a mix thereof,
- LlF is selected from avian LIF or mammalian LIF, or a mix thereof,
- IGF-1 is selected from avian IGF-1 or mammalian IGF-1, or a mix thereof, or
- SCF is selected from avian SCF or mammalian SCF, or a mix thereof.

4. The process for isolating embryonic stem cells (100) according to any one of claims 1 to 3 further comprising at least one inhibitor leading to the inhibition of PKC-signalling.

5. The process for isolating embryonic stem cells (100) according to any one of preceding claims wherein :
- the inhibitor of MEK-signalling is selected from a small molecule or a MEK-1 siRNA,
- the inhibitor of Wnt-signalling is selected from a small molecule or a Wnt-1 siRNA, or
- the inhibitor of PKC- signalling, when present, is selected from a small molecule or a PKC-alpha siRNA.

6. The process for isolating embryonic stem cells (100) according to any one of preceding claims wherein :
- the inhibitor leading to the inhibition of MEK-signalling is selected from : binimetinib, cobimetinib, selumetinib, trametinib, CI-1040, PD035901, TAK-733, PD-325901, PD98059 or U0126 or a mix thereof,
- the inhibitor leading to the inhibition of Wnt-signalling is selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939, or RXC004 or a mix thereof, or
- the inhibitor leading to the inhibition of PKC-signalling, when present, is selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin, or midostaurin, or a mix thereof.

7. The process for isolating embryonic stem cells (100) according to any one of preceding claims wherein the animal serum substitute, is selected from : KnockOut^{™} serum replacement, Serum Replacement Basic ; Serum Replacement 3 ; BIT 9500 Serum Substitute from StemCell.

8. The process for isolating embryonic stem cells (100) according to any one of preceding claims wherein avian embryonic cells are chicken embryonic stem cells or duck embryonic stem cells.

9. A process for obtaining a continuous diploid cell line (200) derived from avian embryonic stem cells (ESCs) comprising :
a. providing (201) at least one ESC obtained from the process (100) for isolating embryonic stem cells from at least one avian embryo according to any one of claims 1-8,
b. progressively withdrawing each of growth factors from culture medium (203), progressively withdrawing from the at least one inhibitor culture medium (204), progressively decreasing the concentration of feeder cells in the culture (205) so as to obtain a total withdrawal of feeder cells after several passages,
thereby obtaining adherent or non-adherent continuous diploid avian cell lines derived from non-human ESC capable of proliferating in a basal medium in the absence of growth factors.

10. A kit of part adapted for reconstituting a cell culture medium comprising:
- an ortholog of interleukin 6 (IL6),
- an ortholog of Leukemia inhibitory factor (LIF),
- an ortholog of Insulin Growth factor 1 (IGF-1),
- an ortholog of Stem Cell Factor (SCF),
- at least one inhibitor of MEK signalling selected from i) a small molecule selected from binimetinib, cobimetinib, selumetinib, trametinib, CI-1040, PD035901, TAK-733, PD-325901, PD98059 or U0126, or a mix thereof, or ii) a MEK-1 siRNA,
- at least one inhibitor of Wnt signalling is selected from i) a small molecule selected from adavivint, capmatinib, CCT251545, FH535, ginsenoside Rh4, ICG-001, iCRT14, IQ-1, isoquercitrin, IWP-2, IWP-4, IWR-1-endo, JW55, KY02111, KY-05009, KYA1797K, lanatoside C, LF3, M2912, M435-1279, MSAB, NCB-0846, PNU-74654, PRI-724, prodigiosin, RCM-1, resibufogenin, salinomycin, triptonide, WIKI4, XAV-939 or RXC004, or a mix thereof or ii) a Wnt-1 siRNA.

11. The kit according to claim 10 which further comprises at least one inhibitor of PKC signalling selected from i) a small molecule selected from Gö6983, Gö6976, enzastaurin, ruboxistaurin, staurosporine, GF 109203X (bisindolylmaleimide I), ZIP, Ro 31-8220, Ro 32-0432, sotrastaurin, rottierin, K252a, bisindolylmaleimide II, calphostin C, chelerythrine, L-threo dihydrosphingosine, melittin or midostaurin or a combination thereof or ii) a PKC-alpha siRNA.

12. The kit according to anyone of claims 10 or 11 further comprising animal serum substitute.
